# EUROPEAN PATENT SPECIFICATION

(11) **EP 2 903 520 B1**
(45) Date of publication and mention of the grant of the patent: **18.04.2018**
(21) Application number: 13802438.5
(22) Date of filing: 26.09.2013
(51) Int. Cl.: A61B 6/00

(54) **VISUALIZING IMAGE DATA**
VISUALISIERUNG VON BILDDATEN
VISUALISATION DE DONNÉES D'IMAGE

(30) Priority: 01.10.2012 US 201261708331 P
(43) Date of publication of application: 12.08.2015
(73) Proprietor: Koninklijke Philips N.V., 5656 AE Eindhoven (NL)
(72) Inventor: PETERS, Joost Frederik, NL-5656 AE Eindhoven (NL); ALGRA, Egbert, NL-5656 AE Eindhoven (NL)
(74) Representative: Zhu, Di
(86) International application number: PCT/IB2013/058884
(87) International publication number: WO 2014/053964

(56) References cited:
- CA-A1- 2 579 858
- US-A1- 2009 003 679
- US-A1- 2009 094 513
- US-A1- 2012 162 222

## Description

### FIELD OF THE INVENTION

The invention relates to a system and method for visualizing image data. The invention further relates to a server comprising the system and to a client device enabling a user to interact with the server. The invention further relates to a computer program product comprising instructions for causing a processor system to perform the method.

Image data may be presented in various ways to a user. For example, in case the image data is 3D image data, the 3D image data may be presented by using a volume rendering technique to generate a 2D projection of the 3D image data. In another example, if the image data is high dynamic range (HDR) image data such as a 2D Computed Tomography (CT) slice, the HDR image data may be presented to the user by using a window level/width rendering to generate a standard dynamic range (SDR) view of the HDR image data. In general, such presentations of the image data are referred to as views of the image data, and said generating of the views of the image data is referred to as view generation. Several of such techniques are known from the field of image processing.

It may be desirable to present a plurality of different views of the image data simultaneously to a user. For example, in case the image data is 3D medical image data of a patient, it may be desirable to simultaneously present different views of the 3D medical image data so as to allow a clinician to quickly obtain an overview of the 3D medical image data. Examples of such views are, e.g., a coronal view, a sagittal view and an axial view. Such a simultaneous presentation of a plurality of different views is henceforth referred to as a gallery of the plurality of different views.

### BACKGROUND OF THE INVENTION

It may be desirable to enable the user to adjust a view generated from the image data. US 2010/0049740 A1 describes a workflow management system for processing medical image data generated by a medical imaging device. It is said that a so-termed scene contains metadata generated from one workflow stage. The metadata in the scene is generated by the image processing operations of the workflow stage, and allows, when applying the scene to the medical image data, to produce a set of medical image views.

When reviewing the medical image views reproduced from the scene, a user may adjust these image views by making updates to the image processing parameters (metadata) contained in the scene. Afterwards, the updated image processing parameters can be saved to the scene to replace the previously stored image processing parameters. The newly updated scene can also be stored in the workflow scene to replace the old scene.

The above system thus allows a user to adjust a particular medical image view by updating the metadata element associated with the medical image view. CA-A-2 579 858 discloses a system for visualising image data comprising the features defined in the preamble of claim 1.

### SUMMARY OF THE INVENTION

A problem of the above system is that it is too inconvenient for a user to obtain a desired view of the image data.

It would be advantageous to provide a system or method which enables a user to more conveniently obtain a desired view of image data.

To better address this concern, a first aspect of the invention provides a system for visualizing image data according to claim 1. In a further aspect of the invention, a method is provided for visualizing image data according to claim 16. In a further aspect of the invention, a computer program product is provided comprising instructions for causing a processor system to perform the method set forth.

The aforementioned measures enable the user to obtain a visualization of the image data by providing the user with a plurality of different views of the image data. For that purpose, the image data is accessed, e.g., from an internal or external location. Several presentations of the image data are generated, thereby obtaining a plurality of different views. Each view is defined at least in part by one or more view parameters. The views differ from each other, e.g., by having different view parameter values and/or by being generated from different parts of the image data, e.g., from different objects comprised in the image data. The plurality of different views is simultaneously presented by being included in a gallery.

The user can select one of the different views from the gallery, e.g., by clicking on the respective view with an input device. The user can adjust the selected view by adjusting one of the view parameters. As a result, the selected view is adjusted, thereby obtaining an at least somewhat different view of the image data. Based on the adjusting of the selected view by the user, at least another one of the views is adjusted automatically. The gallery is then updated to reflect the selected view being adjusted by the user and the other view(s) being adjusted automatically. Hence, the user is presented with an updated gallery.

The aforementioned measures have the effect that the user is presented with gallery comprising a plurality of different views of the image data. Advantageously, compared to showing a single view of the image data, it is more likely that the user is already shown with a view that corresponds to a desired view of the image data, or one that forms a satisfactory basis for obtaining the desired view of image data. The user is further enabled to select one of the views and adjust the view to his or her preference by adjusting a view parameter of said view. The adjusted view parameter thus embodies a preference of the user, e.g., leading to the desired view. By then adjusting at least another one of the plurality of different views based on the adjusted view parameter, the user is presented with a gallery that comprises at least a further view that is automatically adjusted based on his or her preference.

The present invention is based on the insight that a large degree of freedom exists in visualizing image data. This typically corresponds to views being defined by large numbers of view parameter, say n view parameters. It is difficult for a user to obtain a desired view of the image data since this corresponds to obtaining a particular set of view parameters within an n-dimensional parameter space constituted by the n view parameters, i.e., constituting an n-dimensional optimization problem for the user. The present invention enables the user to more conveniently obtain the desired view by simultaneously presenting a plurality of different views, by enabling the user to adjust said views, and by automatically adjusting one or more other views in the gallery based on the adjustment. Advantageously, the user can more quickly and/or more conveniently obtain the desired view. Advantageously, the user does not need to individually adjust each of the plurality of different views. Rather, a manual adjustment of one the views results in an automatic adjustment of one or more of the other views in the gallery. Advantageously, a preference as embodied by the adjusted view parameter is automatically applied to other views.

Optionally, the user interface subsystem is arranged for enabling the user to iteratively select and adjust a number of views from of the plurality of different views, and the display processor is arranged for updating the gallery in response to each adjusting of one of the number of views. The user can thus obtain a desired view by iteratively manually adjusting a view parameter, with the system, in response, automatically adjusting one or more of the other views in the gallery. Advantageously, the user can more quickly and/or more conveniently obtain the desired view.

Optionally, the display processor is arranged for adjusting said other view by i) adjusting a view parameter of the other view based on the adjusted view parameter, thereby obtaining a further adjusted view parameter, and ii) generating the adjusted other view based on the further adjusted view parameter. The other view is thus adjusted by adjusting a view parameter of the other view. Advantageously, the manner of adjusting the other view by the system matches the manner of adjusting the selected view by the user.

Optionally, the view parameter of the other view and the adjusted view parameter differ in type. In response to the user adjusting a certain type of view parameter, the system thus adjusts the other view by adjusting a different type of view parameter. This aspect of the present invention is based on the insight that an adjustment of a type of view parameter of the selected view may necessitate or desire a change in a different type of view parameter of the other view. A reason for this is that the other view may not have a same type of view parameter. In this case, the system is nevertheless able to adjust the other view by adjusting a different type of view parameter. Moreover, even if the other view has a same or similar type of view parameter, it may nevertheless be desirable to adjust a different type of view parameter, e.g., due to the views showing different images and thus resulting in the user having different visualization needs for said images.

Optionally, the display processor is arranged for selecting the view parameter of the other view amongst a plurality of view parameters of the other view based on a similarity in type to the adjusted view parameter. The similarity in type is thus taken into account when selecting the view parameter of the other view, e.g., by selecting a view parameter of the other view that is most similar to the adjusted view parameter.

Optionally, the display processor is arranged for converting a value of the adjusted view parameter to a value of the view parameter of the other view based on a difference in type between the adjusted view parameter and the view parameter of the other view. An adjustment of a value of a type of view parameter may necessitate or desire a different adjustment of a value of a different type of view parameter. By converting the value of the adjusted parameter to a value of the view parameter of the other view based on said difference in type, said necessary or desired different adjustments can be effected.

Optionally, the adjusted view parameter and/or the further adjusted view parameter is one of the group of:
- a view position;
- a view orientation;
- a rendering parameter;
- a projection parameter;
- a slab thickness;
- a zoom factor;
- a brightness or window level; and
- a contrast or window width.

Optionally, the display processor is arranged for omitting adjusting the view parameter of the other view if said view parameter is indicated as a non-adjustable view parameter. It is therefore possible to fix certain view parameters in that they are not automatically adjusted in response to the adjustment of the view parameter by the user.

Optionally, the gallery is constituted by views having a first display quality, and the display processor is arranged for displaying the selected view at a second display quality during the adjusting of the view parameter, with the second display quality being higher than the first display quality. The gallery effectively constitutes a gallery of previews, and the user is enabled to adjust a selected one of the plurality of previews while it is presented at a higher display quality, e.g., constituting a non-preview version of said view.

Optionally, the user interface subsystem is arranged for enabling the user to adjust the view parameter by i) causing the display processor to generate a further gallery comprising variants of the selected view which are generated based on different values of the view parameter, and ii) enabling the user to establish the adjusted view parameter by selecting one of the variants of the selected view from the further gallery. Advantageously, an intuitive and consistent user interface is provided to the user in that a manner of selecting a view for adjustment and the actual adjustment of the view parameter of the selected view are similar or the same since they are both based on selecting a view from a gallery.

Optionally, the display processor is arranged for i) bookmarking one or more views of the updated gallery by generating bookmark data comprising view parameters defining said one or more views, and/or ii) generating one or more of the plurality of different views of the gallery based on bookmark data comprising view parameters defining said one or more views.

Optionally, the display processor is arranged for generating the plurality of different views based on at least one of: a user identifier identifying the user, metadata of the user, usage history information, metadata of the image data, and bookmarked views associated with the image data.

Optionally, the image interface is arranged for accessing the image data from a Picture Archiving and Communication System.

Optionally, a server is provided comprising the system set forth, wherein the user interface subsystem is connectable to a client device, the client device comprising a display and a user input for enabling the user to interact with the server, wherein the user interface subsystem is arranged for i) providing display data of the display processor to the display of the client device, and ii) receiving user data from the user input of client device.

Optionally, a client device is provided comprising a display and a user input for enabling the user to interact with the server set forth.

Optionally, the client device is constituted by a tablet device or Smartphone.

It will be appreciated by those skilled in the art that two or more of the above-mentioned embodiments, implementations, and/or aspects of the invention may be combined in any way deemed useful.

Modifications and variations of the method, the computer program product, the server and/or the client device, which correspond to the described modifications and variations of the system, can be carried out by a person skilled in the art on the basis of the present description.

A person skilled in the art will appreciate that the method may be applied to multi-dimensional image data, e.g. to two-dimensional (2-D), three-dimensional (3-D) or four-dimensional (4-D) images. A dimension of the multi-dimensional image data may relate to time. For example, a three-dimensional image may comprise a time domain series of two-dimensional images. The image may be acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

The invention is defined in the independent claims. Advantageous yet optional embodiments are defined in the dependent claims.

### BRIEF DESCRIPTION OF THE DRAWINGS

These and other aspects of the invention are apparent from and will be elucidated with reference to the embodiments described hereinafter. In the drawings,
Fig. 1 shows a system for visualizing image data, the system being connected to a client device for enabling a user to interact with the system;
Fig. 2 shows a method for visualizing image data;
Fig. 3 shows a computer program product for visualizing image data;
Fig. 4 shows a tablet device comprising a touch sensitive display for enabling a user to interactively select one of a plurality of different views from a gallery;
Fig. 5a shows an example of a gallery which comprises a plurality of different views from medical image data of a patient;
Fig. 5b shows a top-left one of said plurality of views having been selected;
Fig. 5c shows the selected view having been adjusted based on an adjustment of a zoom parameter, a pan parameter and a geometry position parameter;
Fig. 5d shows the adjusted selected view being further adjusted in contrast based on a selection from a further gallery comprising variants of the adjusted selected view;
Fig. 5e shows a top-right one of said views having been selected; and
Fig. 5f shows an updated gallery in which a plurality of other views has been automatically adjusted based on the adjusted selected view.

### DETAILED DESCRIPTION OF EMBODIMENTS

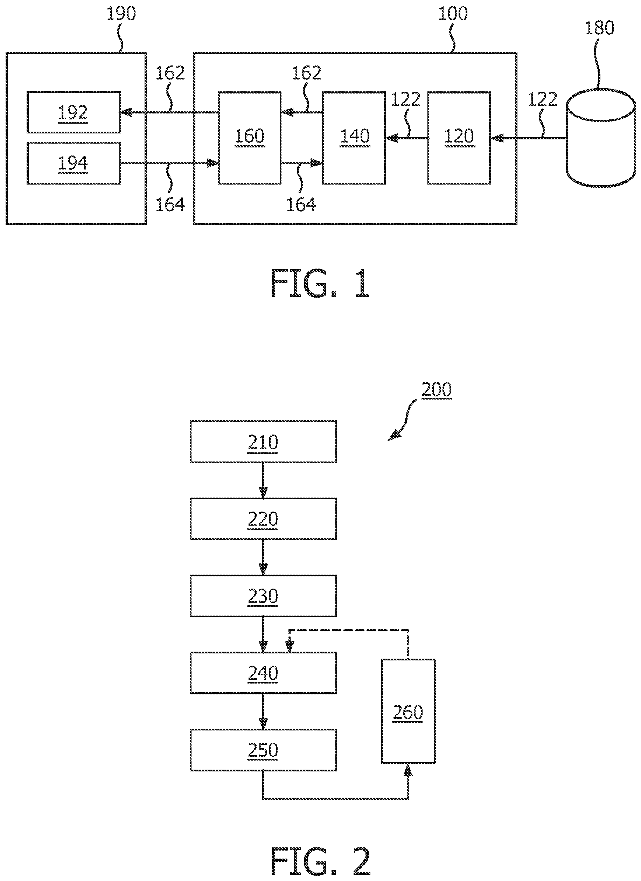

Fig. 1 shows a system 100 for visualizing image data 122. The system 100 comprises an image interface 120 for accessing the image data 122. Fig. 1 shows the system accessing the image data 122 from an external storage 180. Alternatively, the image data 122 may be accessed internally within the system 100, i.e., from an internal storage. The system 100 further comprises a display processor 140. The display processor 140 is shown to receive the image data 122 through the image interface 120. The system 100 further comprises a user interface subsystem 160. The user interface subsystem 160 is shown to receive display data 162 from the display processor 140, the display data constituting a data representation of displayable output of the display processor 140. The user interface subsystem 160 is shown to provide the display data 162 to a display 192 for displaying the displayable output of the display processor 140 to a user. The user interface subsystem 160 is further shown to receive user data 164 from a user input 194 constituting input from the user.

Fig. 1 shows the user interface subsystem 160 being connected to an external client device 190 which comprises the display 192 and the user input 194. In this example, the system 100 may constitute a server 100 which together with the client device 190 forms a client-server system. Alternatively, the display 192 and the user input 194 may each be constituted by separate devices. Alternatively, the system 100 may comprise the display 192 and the user input 194, i.e., both may be an integral part of the system 100.

The external storage 180 may be a Picture Archiving and Communication System (PACS). The system 100 and the PACS 180 may both be part of a Hospital Information System (HIS). The client device may be connectable to the HIS.

An operation of the system 100 may be briefly explained as follows. The display processor 140 receives the image data 122 and generates a plurality of different views of the image data based on one or more view parameters defining each one of the plurality of different views. The display processor further generates a gallery comprising the plurality of different views. The gallery is provided, possibly as part of other displayable output, to the user interface subsystem 160 in the form of display data 162. The user interface subsystem 160 provides the display data 162 to the display 192 for display to the user. The user interface subsystem 160 further receives user data 164 from the user input 194 to enable the user to interact with the system 100 based on the display of the display data 162.

Accordingly, the user can select one the plurality of different views from the gallery. As a result, a selected view is obtained. The user can then adjust the selected view by adjusting a view parameter of the selected view. As a result, an adjusted view parameter is obtained. In response, the display processor 140 updates the gallery by adjusting at least another one of the plurality of different views based on the adjusted view parameter. As a result, an updated gallery is obtained comprising an adjusted other view.

Fig. 2 shows a method 200 for visualizing image data. The method 200 may correspond to an operation of the system 100, e.g., the aforementioned operation. However, it is noted that the method 200 may also be performed in separation of the system 100.

The method 200 comprises, in a first step titled "ACCESSING IMAGE DATA", accessing 210 the image data. The method 200 further comprises, in a second step titled "GENERATING PLURALITY OF VIEWS", generating 220 a plurality of different views of the image data based on one or more view parameters defining each one of the plurality of different views. The method 200 further comprises, in a third step titled "GENERATING GALLERY", generating 230 a gallery comprising the plurality of different views. The method 200 further comprises, in a fourth step titled "USER SELECTING VIEW", enabling 240 the user to select one the plurality of different views from the gallery, thereby obtaining a selected view. The method 200 further comprises, in a fifth step titled "USER ADJUSTING VIEW", enabling 250 the user to adjust the selected view by adjusting a view parameter of the selected view, thereby obtaining an adjusted view parameter. The method 200 further comprises, in a step titled "ADJUSTING OTHER VIEW AND UPDATING GALLERY", updating 260 the gallery by adjusting at least another one of the plurality of different views based on the adjusted view parameter, thereby establishing an adjusted other view in the gallery. Fig. 2 further shows, by means of a dashed line, the method 200 being optionally performed in an iterative manner, indicating that the method 200 may again continue with the fourth step of enabling 240 the user to select one the plurality of different views from the updated gallery, thereby obtaining a selected view, etc.

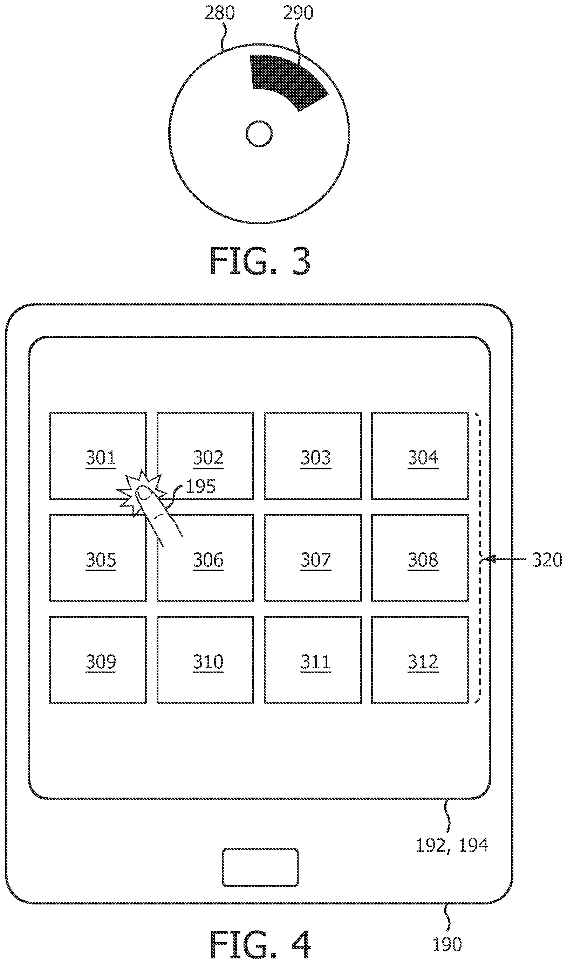

Fig. 3 shows a computer program product 290 comprising instructions for causing a processor system to perform the aforementioned method 200. The computer program product 290 may be comprised on a computer readable medium 280, for example in the form of as a series of machine readable physical marks and/or as a series of elements having different electrical, e.g., magnetic, or optical properties or values.

Fig. 4 schematically illustrates a gallery 320 comprising a plurality of different views 301-312. The gallery 320 shows the plurality of different views 301-312 in a grid-like format. However, this is not a limitation. In the example of Fig. 4, the gallery 320 is displayed on a touch sensitive display 192, 194 of a tablet device 190, with the tablet device constituting an exemplary embodiment of the client device 190. The touch sensitive display 192, 194 provides the functionality of both display 192 as well as user input 194. The user can provide user input 164 by touching 195 the touch sensitive display 192, 194. Accordingly, the user can interactively select one of the plurality of different views 301-312 from the gallery 320 by touching said view. The user interface subsystem may comprise a web application viewable in a browser of the client device 190, or may be connectable to a so-termed native application executable on the client device 190. Hence, upon execution of the web application or the native application on the client device 190, the user may be presented with a user interface with enables the user to interact with the system 100.

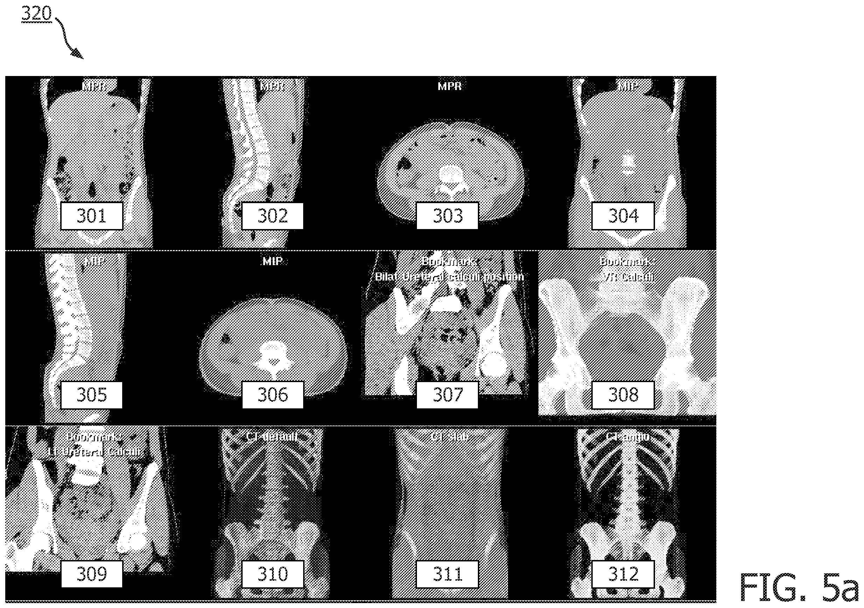

The operation of the system 100 and the method 200 may be explained in more detail as follows. Fig. 5a shows an example of a gallery 320 which comprises a plurality of different views 301-312 from medical image data of a patient. It is noted that in the following, each of the plurality of different views 301-312 are also referred to by number, with the numbering being sequentially from left to right and from top to bottom, i.e., a first view 301 denoting a top-left view and a twelfth view 312 denoting a bottom-right view.

In this particular example, the different views are obtained from a CT image acquisition which provided image data comprising a thousand 2D slices. The plurality of views 301-312 differ in various aspects, including visualization type (Multi-Planer Reformatting (MPR), Maximum Intensity Projection (MIP), and Surface Volume Rendering (SVR)), patient orientation (Coronal, Sagittal, Axial) and slab thickness (2mm, 15 mm, full volume). More specifically, the first view 301 corresponds to a coronal MPR view, the second view 302 to sagittal MPR view, the third view 303 to an axial MPR view, the fourth view 304 to a coronal MIP view, the fifth view 305 to a sagittal MIP view, and the sixth view 306 to an axial MIP view. The plurality of different views 301-312 also includes a seventh view 307, an eight view 308 and a ninth view 309 constituting bookmarked views 307-309 which correspond to views of the image data which were generated earlier in time and then bookmarked by the system for later viewing. Said bookmarked views may correspond to adjusted views, i.e., as manually adjusted by the user or automatically adjusted by the system. Moreover, a tenth view 310, an eleventh view and a twelfth view 312 correspond to coronal volume rendered views with different Look-Up Tables (LUTs) for converting Hounsfield Unit (HU) values to color and opacity values, with each LUT being optimized for presenting different anatomies.

The gallery 320 shown in Fig. 5a may constitute an initial gallery 320 in that it comprises a plurality of different views 301-312 which may be automatically established by the system 100, e.g., in response to a request from the user. The display processor 140 may be arranged for automatically establishing view parameters defining each of the plurality of views 301-312. For that purpose, the display processor 140 may use, e.g., a user identifier. For example, if the user identifier indicates that the user is a radiologist, the plurality of views 301-312 may be established such that they are suitable for viewing by the radiologist. Alternatively or additionally, the display processor 140 may use metadata of the user, with the user being identified by the user identifier or using separate means. The metadata may provide contextual information of the user, e.g., a group membership of the user, a role of the user, preferences of the user such as viewing preferences.

Alternatively or additionally, the display processor 140 may use metadata of the image data 122 which may provide contextual information of the image data, e.g., a procedure code, a body part, an acquisition modality or parameter, etc. The metadata may be included in the image data 122, e.g., by means of DICOM attributes, or may be associated with the image data 122 through another mechanism, e.g., associated HL7 messages relating to a same patient or study. The metadata may also be associated with the user or the system 100 itself. Alternatively or additionally, contextual information of a user interface which is provided on the client device may be used. For example, if a presentation of the gallery 320 is requested by the user using a Graphical User Interface (GUI) element which is associated with contrast/brightness, the display processor 140 may establish a gallery 320 comprising a plurality of views which each are generated based on different contrast/brightness parameters. Alternatively or additionally, existing workflow results may be used which are associated with the image data 122. For example, bookmarked views which were generated from the image data 122 at an earlier stage in a workflow are likely of high interest for a user at a later stage in the workflow. Alternatively or additionally, usage history information may be used which may be indicative of, e.g., historic actions of the user or other users in a similar context. Alternatively or additionally, view parameters of an initial 3D presentation of the image data may be used. For example, a zoom and view orientation in the initial 3D presentation of the image data may be used to establish similar view parameters of the plurality of views 301-312 when the user requests presentation of a gallery 320.

The above contextual information, i.e., the user identifier identifying the user, the metadata of the user, the usage history information, the metadata of the image data 122, and the bookmarked views associated with the image data, may also be used to rank the individual views within the gallery, e.g., to present a most relevant view as the first view 301 and a least relevant view as the twelfth view 312. It is noted that, although not shown in Fig. 1, the system may comprise an interface for receiving the contextual information. For example, the metadata of the image data 122 may be received by the image interface 120 from the external storage 180. Similarly, the metadata of the user may be received by a further interface of the system 100, e.g., from an administrative system or a PACS.

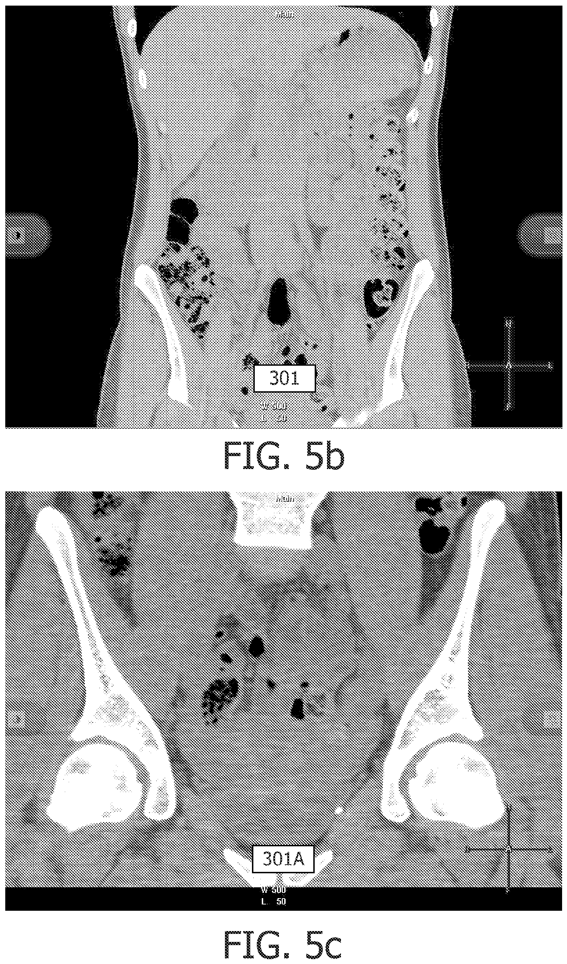

The user may select one of the plurality of different views 301-312 from the gallery 320. In the example of Fig. 5a, the user selects a first view 301 from the gallery 320. In response, the user is enabled to adjust the selected view 301 by adjusting a view parameter of the selected view 301. The gallery 320 may be constituted by previews 301-312, e.g., views having a relatively low resolution, or in general, relatively low display quality. The display processor 140 may be arranged for, upon the user selecting one of the plurality of different views 301-319, displaying the selected view 301 at a higher resolution, or in general, at a higher display quality, than the preview of the selected view 301. While adjusting the view parameter, the selected view 301 may be displayed instead of the gallery 320 or simultaneously with the gallery, e.g., next to the gallery 320 or on a different display. Fig. 5b shows an example showing the first view 301 at a higher resolution. The user may now adjust the selected view 301, e.g., using GUI elements (not shown in Fig. 5b), keyboard buttons, touch, etc. The display processor 140 may be arranged for adjusting the selected view 301 in real-time so as to provide the user with feedback on the adjustment. Fig. 5c shows a result of the user adjusting the first view 301 by adjusting a zoom parameter, a pan parameter and a geometry position parameter. As a consequence, the first view 301 is adjusted, thereby obtaining an adjusted first view 301A. The display processor 140 may, e.g., after an indication of the user that he/she is finished adjusting the selected view 301, update the gallery 322 by replacing the first view 301 with the adjusted first view 301A and by adjusting at least another one of the plurality of different views based on the adjusted view parameter.

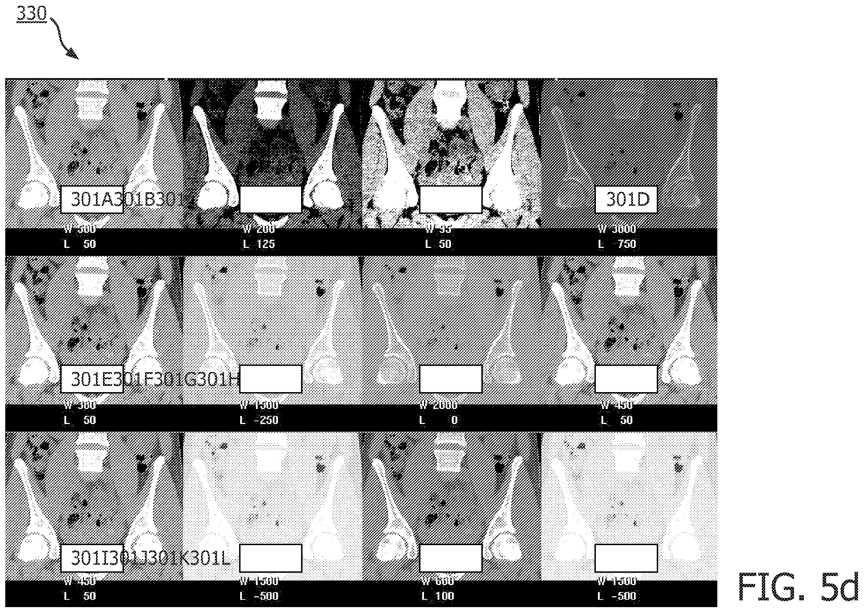

Fig. 5d shows an optional aspect of the present invention in that the user may adjust a view parameter by means of a further gallery 330. For that purpose, the user interface subsystem 160 may be arranged for enabling the user to adjust the view parameter by causing the display processor 140 to generate a further gallery 330 comprising variants of the selected view which are generated based on different values of the view parameter.

In the example of Fig. 5d, after adjusting the zoom parameter, the pan parameter and the geometry position parameter of the first view 301, and thereby obtaining the adjusted first view 301A, the user may further adjust brightness and contrast parameters of said view 301A by means on the aforementioned further gallery 330. In response, the display processor 140 may generate the further gallery 330 comprising the adjusted first view 301A at different brightness/contrast parameter combinations. In this respect, it is noted that the brightness and contrast parameters are here referred to as window level and window width parameters, as is customary in the field of medical imaging. The different window level/window width parameter combinations are indicated in Fig. 5d using the abbreviation 'W' for window width and 'L' for window level. The further gallery 330 comprises as a first view the adjusted first view 301A without further window level/window width adjustment. The further views 301B-301L constitute a same view of the image data, i.e., same zoom parameter, pan parameter and geometry position parameter, but having different window level/window width parameter combinations. The user can thus immediately see the effect of the various adjustments as applied to the adjusted first view 301A from the further gallery 330. The user interface subsystem 160 may be further arranged for enabling the user to establish the adjusted view parameter by selecting one of the variants of the selected view from the further gallery 330. In the example of Fig. 5d, the user selects a fourth view 301D, thus selecting a value of 3000 for window width and a value of 750 for the window level.

In general, the further gallery 330 constitutes an addition or alternative to other means of adjustments, e.g., using GUI elements or keyboard buttons to vary view parameters. The further gallery 330 may therefore also be used to adjust the aforementioned zoom parameter, pan parameter, geometry position parameter, etc, of the first image 301 so as to obtain the adjusted first image 301A. It is noted that the different view parameters, i.e., the different variants, may be established automatically by the system based on, e.g., medical image viewing protocols. In general, such variants may also be obtained by, e.g., automatically varying a value of the view parameter (non-)uniformly across a range.

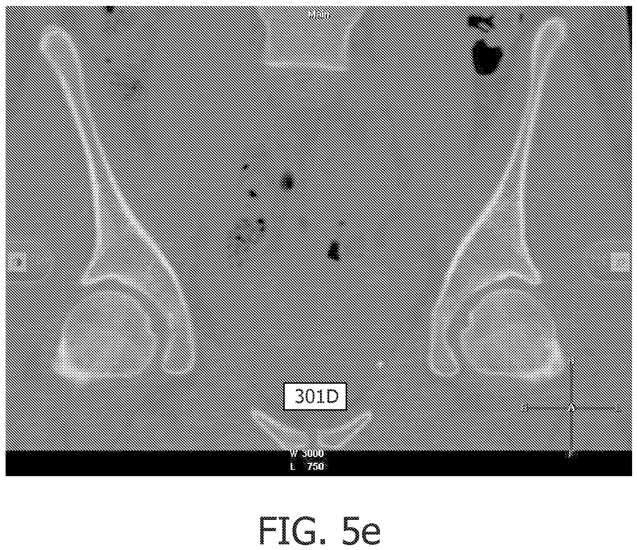

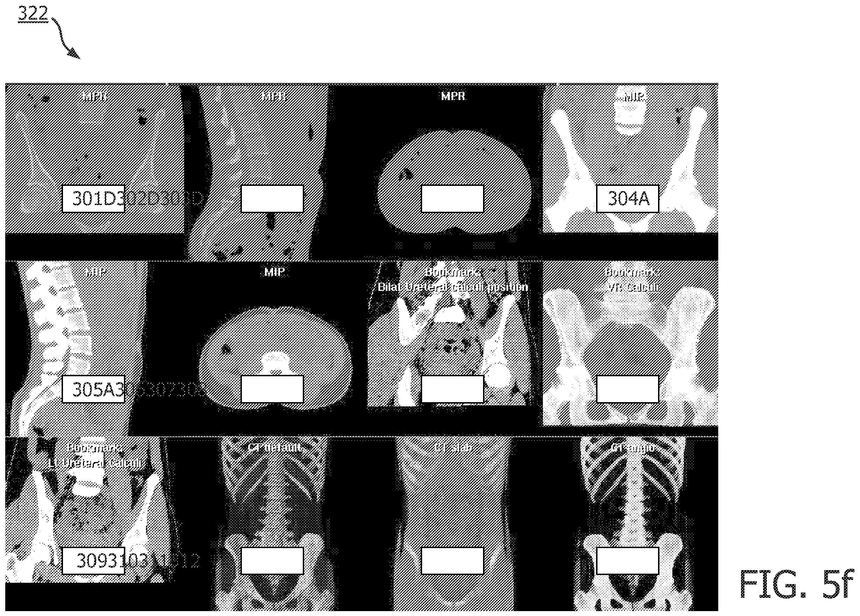

In response to the user selecting the fourth view 301D from the further gallery 330, the display processor 140 may display the fourth view 301D at a higher resolution, or in general, at a higher display quality. A reason for this may be to enable the user to closely view the selected view 301D or to further fine-tune the value of the view parameter which corresponds to the selected view 301D. Fig. 5e shows a result of this, showing the fourth view 301D at a higher resolution. The display processor 140 may then, e.g., after an indication of the user that he/she is finished viewing or adjusting the selected view 301D, update the gallery 322 by replacing the first view 301 with the fourth view 301D from the further gallery 330 and by adjusting at least another one of the plurality of different views based on the adjusted view parameter. Fig. 5f shows a result of this. Here, the second view 302, the fourth view 304 and the fifth view 305 have each been adjusted based on the aforementioned adjustment of the zoom parameter, the pan parameter and the geometry position parameter of the first view 301. As a result, the adjusted second view 302D, the adjusted fourth view 304A and the adjusted fifth view 305A also show a zoomed-in and panned view. Moreover, the second view 302 and the third view 303 have each been adjusted based on the aforementioned adjustment of the window level/window width parameters of the adjusted first view 301A, i.e., based on the window level/window width parameters of the fourth view 301D of the further gallery 330. In this particular example, the window level/window width parameters of the second view 302 and the third view 303 are adjusted so as to be the same as those of the fourth view 301D. Hence, the adjusted second view 302D and the adjusted third view 303D have a same window level/window width appearance, i.e., same brightness/contrast, as the fourth view 301D of the further gallery 330.

In general, the view parameter of the other view and the adjusted view parameter may differ in type. For example, if the user adjusts an opacity parameter of a MIP view, the display processor 140 may adjust a LUT constituting a view parameter of a volume rendering of another view. The display processor 140 may be arranged for selecting the view parameter of the other view amongst a plurality of view parameters of the other view based on a similarity in type to the adjusted view parameter. For example, the display processor 140 may select a conceptually most similar view parameter of the other view. The display processor 140 may also be arranged for converting a value of the adjusted view parameter to a value of the view parameter of the other view based on a difference in type between the adjusted view parameter and the view parameter of the other view. For that purpose, the display processor 140 may use conversion data which indicates how to convert values between different types of view parameters. Examples of view parameters are a view position and a view orientation, e.g., in case the image data is 3D image data. Another term for the combination of view position and view orientation is geometry position. Other examples are a rendering parameter, e.g., in case the view is generated using a volume rendering technique, a projection parameter, e.g., in case the view is generated using a projection technique, a zoom factor, a brightness or window level, and a contrast or window width. Other examples of view parameters are slab thickness, view rotation, view roll, or so-termed render region. The latter may comprise values such as 'full', 'slab' or 'beyond', indicating an extent of the rendering orthogonal to the viewing plane, i.e., along the view direction. The display processor 140 may be arranged for omitting adjusting the view parameter of the other view if said view parameter is indicated as a non-adjustable view parameter. For example, a geometry position of a sagittal view may be fixed. Hence, although other view parameters of the sagittal view may in principle be adjusted, its geometry position is not adjusted.

The user interface subsystem 160 may arranged for enabling the user to iteratively select and adjust a number of views from the plurality of different views, and the display processor 140 may be arranged for updating the gallery in response to each adjusting of one of the number of views. The user may thus adjust further views of the updated gallery 322 by repeating a similar process as described in reference to Figs. 5a-5e.

It will be appreciated that the present invention enables a user to obtain a desired view of image data based on a What You See Is What You Get (WYSIWYG) gallery of alternative presentations, i.e., different views, of the image data. Since the gallery shows the different views simultaneously, with typically one of the different views being already close to a desired view, there is no need for the user to 'guess' which adjustments are most effective to reach a desired view. The gallery may be advantageously used on client devices such as tablet devices which comprise a touch interface and lack convenient mouse and/or keyboard input. It is noted that the WYSIWYG gallery selection may be an iterative process, in which successive galleries show refinements of previously selected and possibly adjusted views. In particular, the view parameters already determined in earlier steps, either by means of the further gallery-based selection or other means of interacting with the visualization, are taken into account when presenting the next gallery.

It is noted that the present invention may be to medical image data as well as non-medical image data. An example of the latter is geographical image data.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing step of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a storage medium, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for visualizing image data (122), comprising:
- an image interface (120) for accessing the image data (122);
- a display processor (140) for i) generating a plurality of different views (301-312) of the image data based on one or more view parameters defining each one of the plurality of different views, and ii) generating a gallery (320) comprising the plurality of different views; and
- a user interface subsystem (160) for j) enabling the user to select one the plurality of different views (301-312) from the gallery (320), thereby obtaining a selected view (301), and jj) enabling the user to adjust the selected view (301A) by adjusting a view parameter of the selected view, thereby obtaining an adjusted view parameter;
- wherein the display processor (140) is arranged for updating the gallery (322) by adjusting at least another one of the plurality of different views (304, 305) based on the adjusted view parameter, thereby establishing an adjusted other view (304A, 305A) in the gallery;
- **characterised in that** the user interface subsystem (160) is arranged for enabling the user to adjust the view parameter by i) causing the display processor (140) to generate a further gallery (330) comprising variants of the selected view (301A-301L) which are generated based on different values of the view parameter, and ii) enabling the user to establish the adjusted view parameter by selecting one of the variants (301D) of the selected view from the further gallery.

2. The system (100) according to claim 1, wherein the user interface subsystem (160) is arranged for enabling the user to iteratively select and adjust a number of views from of the plurality of different views (301-312), and wherein the display processor (140) is arranged for updating the gallery (322) in response to each adjusting of one of the number of views.

3. The system (100) according to claim 1, wherein the display processor (140) is arranged for adjusting said other view (304, 305) by i) adjusting a view parameter of the other view based on the adjusted view parameter, thereby obtaining a further adjusted view parameter, and ii) generating the adjusted other view (304A, 305A) based on the further adjusted view parameter.

4. The system (100) according to claim 3, wherein the view parameter of the other view and the adjusted view parameter differ in type.

5. The system (100) according to claim 4, wherein the display processor (140) is arranged for selecting the view parameter of the other view (304, 305) amongst a plurality of view parameters of the other view based on a similarity in type to the adjusted view parameter.

6. The system (100) according to claim 4, wherein the display processor (140) is arranged for converting a value of the adjusted view parameter to a value of the view parameter of the other view (304, 305) based on a difference in type between the adjusted view parameter and the view parameter of the other view.

7. The system (100) according to claim 3, wherein the adjusted view parameter and/or the further adjusted view parameter is one of the group of:
- a view position;
- a view orientation;
- a rendering parameter;
- a projection parameter;
- a slab thickness;
- a zoom factor;
- a brightness or window level; and
- a contrast or window width.

8. The system (100) according to claim 3, wherein the display processor (140) is arranged for omitting adjusting the view parameter of the other view (304, 305) if said view parameter is indicated as a non-adjustable view parameter.

9. The system (100) according to claim 1, wherein the gallery (320) is constituted by views (301-312) having a first display quality, and wherein the display processor (140) is arranged for displaying the selected view (301) at a second display quality during the adjusting of the view parameter, with the second display quality being higher than the first display quality.

10. The system (100) according to claim 1, wherein the display processor (140) is arranged for i) bookmarking one or more views of the updated gallery (322) by generating bookmark data comprising view parameters defining said one or more views, and/or ii) generating one or more of the plurality of different views (301-312) of the gallery (320) based on bookmark data comprising view parameters defining said one or more views.

11. The system (100) according to claim 1, wherein the display processor (140) is arranged for generating the plurality of different views (301-312) based on at least one of: a user identifier identifying the user, metadata of the user, usage history information, metadata of the image data (122), and bookmarked views associated with the image data.

12. The system (100) according to claim 1, wherein the image interface (120) is arranged for accessing the image data (122) from a Picture Archiving and Communication System (180).

13. A server comprising the system (100) according to claim 1, wherein the user interface subsystem (160) is connectable to a client device (190), the client device comprising a display (192) and a user input (194) for enabling the user to interact with the server, wherein the user interface subsystem (160) is arranged for i) providing display data (162) of the display processor to the display (192) of the client device, and ii) receiving user data (164) from the user input (194) of client device.

14. A client device (190) comprising a display (192) and a user input (194) for enabling the user to interact with the server according to claim 13.

15. The client device (190) according to claim 14, constituted by a tablet device or Smartphone.

16. A method (200) for visualizing image data, comprising:
- accessing (210) the image data;
- generating (220) a plurality of different views of the image data based on one or more view parameters defining each one of the plurality of different views;
- generating (230) a gallery comprising the plurality of different views;
- enabling (240) the user to select one the plurality of different views from the gallery, thereby obtaining a selected view;
- enabling (250) the user to adjust the selected view by adjusting a view parameter of the selected view, thereby obtaining an adjusted view parameter;
- **characterised by** generating a further gallery (330) comprising variants of the selected view (301A-301L) which are generated based on different values of the view parameter,
- enabling the user to establish the adjusted view parameter by selecting one of the variants (301D) of the selected view from the further gallery;
- updating (260) the gallery by adjusting at least another one of the plurality of different views based on the adjusted view parameter, thereby establishing an adjusted other view in the gallery.

17. A computer program product (290) comprising instructions for causing a processor system to perform the method according to claim 16.

## Patentansprüche

1. System (100) zur Visualisierung von Bilddaten (122), umfassend:
- eine Bildschnittstelle (120) für den Zugriff auf die Bilddaten (122);
- einen Anzeigeprozessor (140) zum i) Erzeugen einer Vielzahl von unterschiedlichen Ansichten (301 - 312) der Bilddaten basierend auf einem oder mehreren Ansichtsparametern, die jede aus der Vielzahl von unterschiedlichen Ansichten definieren, und ii) Erzeugen einer Galerie (320), die die Vielzahl von unterschiedlichen Ansichten umfasst; und
- ein Benutzerschnittstellen-Subsystem (160) zum j) Befähigen des Benutzers, eine der Vielzahl von unterschiedlichen Ansichten (301 - 312) aus der Galerie (320) auszuwählen, wodurch eine ausgewählte Ansicht (301) erhalten wird, und jj) Befähigen des Benutzers, die ausgewählte Ansicht (301A) durch Einstellen eines Ansichtsparameters der ausgewählten Ansicht einzustellen, wodurch ein eingestellter Ansichtsparameter erhalten wird;
- wobei der Anzeigeprozessor (140) angeordnet ist, um die Galerie (322) durch Einstellen von mindestens einer weiteren aus der Vielzahl von unterschiedlichen Ansichten (304, 305) basierend auf dem eingestellten Ansichtsparameter zu aktualisieren, wodurch eine eingestellte weitere Ansicht (304A, 305A) in der Galerie hergestellt wird;
- **dadurch gekennzeichnet, dass** das Benutzerschnittstellen-Subsystem (160) angeordnet ist, um den Benutzer zu befähigen, den Ansichtsparameter einzustellen durch i) Veranlassen des Anzeigeprozessors (140), eine weitere Galerie (330) zu erzeugen, die Abwandlungen der ausgewählten Ansicht (301A - 301L) umfasst, die basierend auf unterschiedlichen Werten des Ansichtsparameters erzeugt werden, und ii) Befähigen des Benutzers, den eingestellten Ansichtsparameter durch Auswählen einer der Abwandlungen (301D) der ausgewählten Ansicht aus der weiteren Galerie herzustellen.

2. System (100) nach Anspruch 1, wobei das Benutzerschnittstellen-Subsystem (160) angeordnet ist, um den Benutzer zu befähigen, eine Anzahl von Ansichten aus der Vielzahl von unterschiedlichen Ansichten (301 - 312) iterativ auszuwählen und einzustellen, und wobei der Anzeigeprozessor (140) angeordnet ist, um die Galerie (322) als Reaktion auf jedes Einstellen von einer aus der Anzahl von Ansichten zu aktualisieren.

3. System (100) nach Anspruch 1, wobei der Anzeigeprozessor (140) angeordnet ist, um die weitere Ansicht (304, 305) einzustellen durch i) Einstellen eines Ansichtsparameters der weiteren Ansicht basierend auf dem eingestellten Ansichtsparameter, wodurch ein weiterer eingestellter Ansichtsparameter erhalten wird, und ii) Erzeugen der eingestellten weiteren Ansicht (304A, 305A) basierend auf dem weiteren eingestellten Ansichtsparameter.

4. System (100) nach Anspruch 3, wobei sich der Ansichtsparameter der weiteren Ansicht und der eingestellte Ansichtsparameter in der Art unterscheiden.

5. System (100) nach Anspruch 4, wobei der Anzeigeprozessor (140) angeordnet ist, um den Ansichtsparameter der weiteren Ansicht (304, 305) unter einer Vielzahl von Ansichtsparametern der weiteren Ansicht basierend auf einer Ähnlichkeit in der Art mit dem eingestellten Ansichtsparameter auszuwählen.

6. System (100) nach Anspruch 4, wobei der Anzeigeprozessor (140) angeordnet ist, um einen Wert des eingestellten Ansichtsparameters in einen Wert des Ansichtsparameters der weiteren Ansicht (304, 305) basierend auf einen Unterschied in der Art zwischen dem eingestellten Ansichtsparameter und dem Ansichtsparameter der weiteren Ansicht umzuwandeln.

7. System (100) nach Anspruch 3, wobei der eingestellte Ansichtsparameter und/oder der weitere eingestellte Ansichtsparameter einer ist aus der Gruppe von:
- einer Ansichtsposition;
- einer Ansichtsausrichtung;
- einem Wiedergabeparameter;
- einem Projektionsparameter;
- einer Scheibendicke;
- einem Zoomvergrößerungsfaktor;
- einer Helligkeit oder Fensterstufe; und
- einem Kontrast oder einer Fensterbreite.

8. System (100) nach Anspruch 3, wobei der Anzeigeprozessor (140) angeordnet ist, um das Einstellen des Ansichtsparameters der weiteren Ansicht (304, 305) wegzulassen, wenn der Ansichtsparameter als nicht-einstellbarer Ansichtsparameter angegeben ist.

9. System (100) nach Anspruch 1, wobei die Galerie (320) von Ansichten (301 - 312) gebildet wird, die eine erste Anzeigequalität aufweisen, und wobei der Anzeigeprozessor (140) angeordnet ist, um die ausgewählte Ansicht (301) in einer zweiten Anzeigequalität anzuzeigen, während der Ansichtsparameter eingestellt wird, wobei die zweite Anzeigequalität höher ist als die erste Anzeigequalität.

10. System (100) nach Anspruch 1, wobei der Anzeigeprozessor (140) angeordnet ist, um i) eine oder mehrere Ansichten der aktualisierten Galerie (322) durch Erzeugen von Lesezeichendaten zu markieren, die Ansichtsparameter umfassen, die die eine oder mehreren Ansichten definieren, und /oder um ii) eine oder mehrere aus der Vielzahl von unterschiedlichen Ansichten (301 - 312) der Galerie (320) basierend auf Lesezeichendaten zu erzeugen, die Ansichtsparameter umfassen, die die eine oder mehreren Ansichten definieren.

11. System (100) nach Anspruch 1, wobei der Anzeigeprozessor (140) angeordnet ist, um die Vielzahl von unterschiedlichen Ansichten (301 - 312) basierend auf mindestens einem aus Folgenden zu erzeugen: einer Benutzerkennung, die den Benutzer identifiziert, Metadaten des Benutzers, Nutzungsverlaufsinformationen, Metadaten der Bilddaten (122) und markierten Ansichten, die zu den Bilddaten zugehörig sind.

12. System (100) nach Anspruch 1, wobei die Bildschnittstelle (120) angeordnet ist, um auf die Bilddaten (122) von einem Bildarchivierungs- und Kommunikationssystem (180) zuzugreifen.

13. Server, umfassend das System (100) nach Anspruch 1, wobei das Benutzerschnittstellen-Subsystem (160) mit einem Client-Gerät (190) verbindbar ist, wobei das Client-Gerät eine Anzeige (192) und eine Benutzereingabe (194) umfasst, um den Benutzer zu befähigen, mit dem Server zu interagieren, wobei das Benutzerschnittstellen-Subsystem (160) angeordnet ist, um i) der Anzeige (192) des Client-Geräts Anzeigedaten (162) des Anzeigeprozessors bereitzustellen und ii) Benutzerdaten (164) von der Benutzereingabe (194) des Client-Geräts zu empfangen.

14. Client-Gerät (190), umfassend eine Anzeige (192) und eine Benutzereingabe (194), um den Benutzer zu befähigen, mit dem Server nach Anspruch 13 zu interagieren.

15. Client-Gerät (190) nach Anspruch 14, das von einem Tablet oder einem Smartphone gebildet wird.

16. Verfahren (200) zur Visualisierung von Bilddaten, umfassend:
- Zugreifen (210) auf die Bilddaten;
- Erzeugen (220) einer Vielzahl von unterschiedlichen Ansichten der Bilddaten basierend auf einem oder mehreren Ansichtsparametern, die jede aus der Vielzahl von unterschiedlichen Ansichten definieren;
- Erzeugen (230) einer Galerie, die die Vielzahl von unterschiedlichen Ansichten umfasst;
- Befähigen (240) des Benutzers, eine der Vielzahl von unterschiedlichen Ansichten aus der Galerie auszuwählen, wodurch eine ausgewählte Ansicht erhalten wird;
- Befähigen (250) des Benutzers, die ausgewählte Ansicht durch Einstellen eines Ansichtsparameters der ausgewählten Ansicht einzustellen, wodurch ein eingestellter Ansichtsparameter erhalten wird;
- **gekennzeichnet durch**
- Erzeugen einer weiteren Galerie (330), die Abwandlungen der ausgewählten Ansicht (301A - 301L) umfasst, die basierend auf unterschiedlichen Werten des Ansichtsparameters erzeugt werden,
- Befähigen des Benutzers, den eingestellten Ansichtsparameter durch Auswählen einer der Abwandlungen (301D) der ausgewählten Ansicht aus der weiteren Galerie herzustellen;
- Aktualisieren (260) der Galerie durch Einstellen von mindestens einer weiteren aus der Vielzahl von unterschiedlichen Ansichten basierend auf dem eingestellten Ansichtsparameter, wodurch eine eingestellte weitere Ansicht in der Galerie hergestellt wird.

17. Computerprogrammprodukt (290), das Befehle umfasst, um ein Prozessorsystem zu veranlassen, das Verfahren nach Anspruch 16 durchzuführen.

## Revendications

1. Système (100) pour visualiser des données d'images (122), comprenant :
- une interface d'images (120) pour accéder aux données d'images (122) ;
- un processeur d'affichage (140) pour i) générer une pluralité de vues différentes (301 à 312) des données d'images sur la base d'un ou plusieurs paramètres de vues définissant chacune de la pluralité de vues différentes, et ii) générer une galerie (320) comprenant la pluralité de vues différentes ; et
- un sous-système d'interface utilisateur (160) pour j) permettre à l'utilisateur de sélectionner une de la pluralité de vues différentes (301 à 312) à partir de la galerie (320), obtenant ainsi une vue sélectionnée (301), et jj) permettre à l'utilisateur d'ajuster la vue sélectionnée (301A) en ajustant un paramètre de vue de la vue sélectionnée, obtenant ainsi un paramètre de vue ajusté ;
- dans lequel le processeur d'affichage (140) est agencé pour mettre à jour la galerie (322) en ajustant au moins une autre de la pluralité de vues différentes (304, 305) sur la base du paramètre de vue ajusté, établissant ainsi une autre vue ajustée (304A, 305A) dans la galerie ;
- **caractérisé en ce que** le sous-système d'interface utilisateur (160) est agencé pour permettre à l'utilisateur d'ajuster le paramètre de vue en i) amenant le processeur d'affichage (140) à générer une galerie supplémentaire (330) comprenant des variantes de la vue sélectionnée (301A à 301L) qui sont générées sur la base de valeurs différentes du paramètre de vue, et ii) permettant à l'utilisateur d'établir le paramètre de vue ajusté en sélectionnant une des variantes (301D) de la vue sélectionnée à partir de la galerie supplémentaire.

2. Système (100) selon la revendication 1, dans lequel le sous-système d'interface utilisateur (160) est agencé pour permettre à l'utilisateur de sélectionner et ajuster de manière itérative un nombre de vues à partir de la pluralité de vues différentes (301 à 312), et dans lequel le processeur d'affichage (140) est agencé pour mettre à jour la galerie (322) en réponse à chaque ajustement de l'une du nombre de vues.

3. Système (100) selon la revendication 1, dans lequel le processeur d'affichage (140) est agencé pour ajuster ladite autre vue (304, 305) en i) ajustant un paramètre de vue de l'autre vue sur la base du paramètre de vue ajusté, obtenant ainsi un paramètre de vue ajusté supplémentaire, et ii) générant l'autre vue ajustée (304A, 305A) sur la base du paramètre de vue ajusté supplémentaire.

4. Système (100) selon la revendication 3, dans lequel le paramètre de vue de l'autre vue et le paramètre de vue ajusté diffèrent en type.

5. Système (100) selon la revendication 4, dans lequel le processeur d'affichage (140) est agencé pour sélectionner le paramètre de vue de l'autre vue (304, 305) parmi une pluralité de paramètres de vue de l'autre vue sur la base d'une similitude de type par rapport au paramètre de vue ajusté.

6. Système (100) selon la revendication 4, dans lequel le processeur d'affichage (140) est agencé pour convertir une valeur du paramètre de vue ajusté en une valeur du paramètre de vue de l'autre vue (304, 305) sur la base d'une différence de type entre le paramètre de vue ajusté et le paramètre de vue de l'autre vue.

7. Système (100) selon la revendication 3, dans lequel le paramètre de vue ajusté et/ou le paramètre de vue ajusté supplémentaire est l'un parmi le groupe constitué de :
- une position de vue ;
- une orientation de vue ;
- un paramètre de rendu ;
- un paramètre de projection ;
- une épaisseur de plaque ;
- un facteur de zoom ;
- une luminosité ou un niveau de fenêtre ; et
- un contraste ou une largeur de fenêtre.

8. Système (100) selon la revendication 3, dans lequel le processeur d'affichage (140) est agencé pour omettre d'ajuster le paramètre de vue de l'autre vue (304, 305) si ledit paramètre de vue est indiqué comme étant un paramètre de vue non ajustable.

9. Système (100) selon la revendication 1, dans lequel la galerie (320) est constituée de vues (301 à 312) ayant une première qualité d'affichage, et dans lequel le processeur d'affichage (140) est agencé pour afficher la vue sélectionnée (301) à une seconde qualité d'affichage pendant l'ajustement du paramètre de vue, la seconde qualité d'affichage étant plus élevée que la première qualité d'affichage.

10. Système (100) selon la revendication 1, dans lequel le processeur d'affichage (140) est agencé pour i) marquer d'un signet une ou plusieurs vues de la galerie mise à jour (322) en générant des données de signet comprenant des paramètres de vue définissant lesdites une ou plusieurs vues, et/ou ii) générer une ou plusieurs de la pluralité de vues différentes (301 à 312) de la galerie (320) sur la base de données de signet comprenant des paramètres de vue définissant lesdites une ou plusieurs vues.

11. Système (100) selon la revendication 1, dans lequel le processeur d'affichage (140) est agencé pour générer la pluralité de vues différentes (301 à 312) sur la base d'au moins un parmi : un identifiant d'utilisateur identifiant l'utilisateur, des métadonnées de l'utilisateur, des informations d'historique d'utilisation, des métadonnées des données d'images (122), et des vues marquées d'un signet associées aux données d'images.

12. Système (100) selon la revendication 1, dans lequel l'interface d'image (120) est agencée pour accéder aux données d'images (122) à partir d'un système d'archivage et de communication d'images (180).

13. Serveur comprenant le système (100) selon la revendication 1, dans lequel le sous-système d'interface utilisateur (160) est connectable à un dispositif client (190), le dispositif client comprenant un affichage (192) et une entrée utilisateur (194) pour permettre à l'utilisateur d'interagir avec le serveur, dans lequel le sous-système d'interface utilisateur (160) est agencé pour i) fournir des données d'affichage (162) du processeur d'affichage de l'affichage (192) du dispositif client, et ii) recevoir des données utilisateur (164) à partir de l'entrée utilisateur (194) du dispositif client.

14. Dispositif client (190) comprenant un affichage (192) et une entrée utilisateur (194) pour permettre à l'utilisateur d'interagir avec le serveur selon la revendication 13.

15. Dispositif client (190) selon la revendication 14, constitué d'un dispositif tablette ou d'un smartphone.

16. Procédé (200) pour visualiser des données d'images, comprenant :
- l'accès (210) aux données d'images ;
- la génération (220) d'une pluralité de vues différentes des données d'images sur la base d'un ou plusieurs paramètres de vue définissant chacune de la pluralité de vues différentes ;
- la génération (230) d'une galerie comprenant la pluralité de vues différentes ;
- la permission (240) pour l'utilisateur de sélectionner une de la pluralité de vues différentes à partir de la galerie, obtenant ainsi une vue sélectionnée ;
- la permission (250) pour l'utilisateur d'ajuster la vue sélectionnée en ajustant un paramètre de vue de la vue sélectionnée, obtenant ainsi un paramètre de vue ajusté ;
- **caractérisé par**
la génération d'une galerie supplémentaire (330) comprenant des variantes de la vue sélectionnée (301A à 301L) qui sont générées sur la base de valeurs différentes du paramètre de vue,
- la permission pour l'utilisateur d'établir le paramètre de vue ajusté en sélectionnant une des variantes (301D) de la vue sélectionnée à partir de la galerie supplémentaire ;
- la mise à jour (260) de la galerie en ajustant au moins une autre de la pluralité de vues différentes sur la base du paramètre de vue ajusté, établissant ainsi une autre vue ajustée dans la galerie.

17. Produit programme d'ordinateur (290) comprenant des instructions pour amener un système de processeur à mettre en oeuvre le procédé selon la revendication 16.
